# EUROPÄISCHE PATENTSCHRIFT

(11) **EP 0 862 903 B1**
(45) Veröffentlichungstag und Bekanntmachung des Hinweises auf die Patenterteilung: **09.04.2003**
(21) Anmeldenummer: 98102995.2
(22) Anmeldetag: 20.02.1998
(51) Int. Cl.: A61F 13/02

(54) **Pflaster mit langnachleuchtendem Aufdruck**
Dressing having a print with long afterglow effect
Pensement comportant une impression lumineuse à longue persistance

(30) Priorität: 08.03.1997 DE 19709606
(43) Veröffentlichungstag der Anmeldung: 09.09.1998
(73) Patentinhaber: Beiersdorf Aktiengesellschaft, 20245 Hamburg (DE)
(72) Erfinder: Leutz, Reiner, 21465 Reinbek (DE); Bruss, Witta, 22605 Hamburg (DE); Bosse, Jürgen, 20253 Hamburg (DE)

(56) Entgegenhaltungen:
- WO-A-94/17766
- BE-A- 1 009 757
- DE-U- 29 518 174
- DE-U- 29 614 078
- GB-A- 2 217 206

## Beschreibung

Die Erfindung betrifft die Weiterentwicklung bekannter Pflaster.

Die Verwendung von Pflastern zur Abdeckung von Wunden ist allgemein angewandte Praxis. Die Pflaster bestehen aus einem Trägermaterial, das auf einer Seite mit einer selbstklebenden Schicht versehen ist. Auf diese selbstklebende Beschichtung ist in den meisten Fällen eine Wundabdeckung aufgebracht. Um eine einfache Handhabung zu gewährleisten, wird die selbstklebende Beschichtung darüber hinaus mit einer schützenden Schicht aus beispielsweise Folie eingedeckt. Daran anschließend werden einzelne Pflaster aus einer Rolle in unterschiedlichen Formen ausgestanzt und in Papier eingesiegelt. Es handelt sich bei den ausgestanzten Formen um geometrisch regelmäßige Formen wie zum Beispiel Rechtecke oder Kreise.

Das deutsche Gebrauchsmuster DE-U-74 20 413 beschreibt eine Plakatplakette mit einer zwei- oder dreidimensionalen Wiedergabe von mindestens einer Kindern bekannten und vorzugsweise bei diesen beliebten figürlichen Darstellung an ihrer Sichtoberfläche, die dadurch gekennzeichnet ist, daß sie als Wundpflaster mit einer das Trägermaterial für die figürliche Darstellung bildenden und auf der Haut zum Haften bringbare Bereiche aufweisenden Deckschicht aus wundfreundlichem und/oder heilungsförderndem und/oder atmungsaktivem Material ausgebildet ist.

Das deutsche Gebrauchsmuster DE-U-296 14 078 beschreibt ein Nasenpflaster mit Werbeaufdruck wobei die Oberfläche phosphoreszierend, selbstleuchtend oder reflektierend dargestellt werden kann.

Bei dieser Art von Darstellungen handelt es sich aber stets um einen Aufdruck der herkömmlichen Art.

Allgemein weisen Pflaster aber überwiegend keinen Aufdruck auf der körperabgewandten Seite des Trägermaterials des Pflasters auf.

Erst seit neuerer Zeit sind vergleichbare Pflaster am Markt anzutreffen. So werden sogenannte Junior-Strips ® mit einem Aufdruck auf einem Pflaster der herkömmlichen Aufmachung vertrieben, das sich insbesondere bei Kindern größter Beliebtheit erfreut. Dieser Aufdruck besteht zumeist aus der Darstellung einer Comicfigur.

Jugendliche und Kinder stehen Pflastern der bekannten Art trotz der unbestrittenen Vorteile bei der Anwendung zur Abdeckung kleinerer Wunden eher ablehnend gegenüber, denn mit dem Pflaster werden negative Erinnerungen wie Schmerz, eventuell Bluten und eine Verletzung verbunden.

Darüber hinaus sehen die herkömmlichen Pflaster unauffällig und damit für Kinder eher unattraktiv aus.

Um den optischen Reiz für Kinder und insbesondere für Jugendliche zu erhöhen, vertreibt die Firma Kendall Futuro unter der Marke Curad ® auf dem US-amerikanischen Markt ein Pflaster mit einer PVC-Folie als Träger. Auf der oberen Seite des Trägermaterials ist ein vollflächiger, mit hoher Wahrscheinlichkeit im gestreuten Flexodruck aufgetragener Aufdruck vorhanden, der nachleuchtende Eigenschaften haben soll. Versuche mit diesem Pflaster haben aber gezeigt, daß der Aufdruck nur äußerst kurzfristig und darüber hinaus nur sehr schwach nachleuchtet.

Weiterhin werden von der Firma Siebdruck Bischoff in Muggensturm langnachleuchtende Sicherheitsleitsysteme und Sicherheitskennzeichen hergestellt. Als Trägermaterialien finden unter anderem selbstklebend beschichtete, 100 µm starke PVC-Folien Verwendung, die im Bogensiebdruck mit einem Lacksystem bedruckt werden, das ein Pigment mit langnachleuchtenden Eigenschaften enthält. Die zur Anwendung kommenden Pigmente basieren dabei auf Selten-Erd-Elementarhaltiger-Erdalkalialuminaten.

Aufgabe der Erfindung war es, insbesondere für Jugendliche und Kinder ein Pflaster zu schaffen, dem die genannten Personengruppen nicht mehr ablehnend gegenüber stehen, sondern das sie sehr interessant finden, und dies mit äußerst einfachen Mitteln. Im speziellen ist die Erfindung auf die Möglichkeit gerichtet, mit Hilfe der erfindungsgemäßen Pflaster offene Wunden an der Hautoberfläche abdecken zu können, um unerwünschte Folgen wie Infektionen zu unterbinden.

Gelöst wird diese Aufgabe durch ein Pflaster, wie es in den Ansprüchen näher beschrieben ist.

Die Pflaster bestehen aus einem Trägermaterial, bevorzugt einer Polyethylenfolie, das auf der oberen Seite vollflächig oder partiell einen langnachleuchtenden Aufdruck aufweist, bestehend aus einem Lacksystem, in das ein langnachleuchtendes Pigment eingearbeitet ist, und das auf der unteren Seite mit einer hautverträglichen selbstklebenden Schicht versehen ist.

Neben den genannten Folien können als Trägermaterial aber alle für die Haut, zum Bedrucken und zum Beschichten mit einer selbstklebenden Masse geeignete Materialien Verwendung finden. Neben Folien eignen sich auch hervorragend Gewebe, Vliesstoffe oder Verbunde aus den genannten Materialien als Träger, solange die an das Trägermaterial gestellten Anforderungen erfüllt sind, und zwar muß der Träger flexibel, bedruckbar und anschmiegsam sein.

Weiterhin ist vorteilhafterweise das Trägermaterial weiß eingefärbt und/oder weiß unterlegt oder bedruckt.

In einer bevorzugten Ausführungsform ist das Pflaster mit einer Wundauflage auf der klebenden Seite des Trägermaterials versehen, wobei die Wundauflage kleiner als die Klebfläche ist und in der Mitte des Trägermaterials aufgebracht ist.

Um die Wundauflage gegen Kontamination und Verschmutzung zu schützen, kann die selbstklebend ausgerüstete Seite des Trägermaterials mit mindestens einem abziehbaren Deckblatt als Schutzabdeckung versehen sein.

In einer weiteren bevorzugten Ausführung stimmt die geometrische Form des Trägermaterials im wesentlichen mit den äußeren Konturen des auf der oberen Seite des Trägermaterials befindlichem Aufdrucks überein.

Als Lacksystem wird vorzugsweise ein transparentes thermisch trocknendes Lacksystem verwandt, beispielsweise erhältlich von der Firma SICPA Druckfarben GmbH, Backnang. Es eignen sich aber auch elektronenstrahlvernetzende Lacksysteme oder UV-Lacksysteme.

In das Lacksystem wird das langnachleuchtende Pigment eingemischt, insbesondere mit einem Mischungsverhältnis von 1:1. Das Pigment wird gegebenenfalls unter Verwendung eines Verdickers so in das Lacksystem eingebracht, daß eine optimale Homogenität und Immobilität gewährleistet ist.

Die Nachleuchtintensität der genannten Mischung ist dabei von der Komgrößenverteilung und der Auftragsmenge auf das Trägermaterial abhängig. Die Auftragsmenge ergibt sich aus der Ausführung des zum Bedrucken zur Verwendung kommenden Siebs.

Bei dem in das Lacksystem eingearbeitete langnachleuchtende Pigment handelt es sich vorzugsweise um eine mit Kupferionen dotierte Zinksulfidverbindung, insbesondere Lumilux ® Effekt grün N-L der Firma Riedel-de Häen. Derartige Pigmente vermögen Energie zu absorbieren und in Form von Lichtquanten zeitlich verzögert wieder abzugeben.

Weiterhin eignen sich allgemein die langnachleuchtenden Pigmente aus der Gruppe Lumilux ® N der Firma Riedel-de Häen, bei denen es sich um polykristalline, anorganische Zink- oder Erdalkalisulfide handelt. Insbesondere letztere, die einen roten oder blauen Nachleuchteffekt bewirken können, müssen dabei durch die Verwendung eines entsprechenden nicht-wäßrigen Binders vor dem direkten Kontakt mit Feuchtigkeit geschützt werden.

Um das Pigment einsetzen zu dürfen, muß das langnachleuchtende Pigment den Anforderungen eines Medizinprodukts erfüllen.

Nach DIN 53160 dürfen kein Farbstoff und kein optischer Aufheller aus dem jeweiligen Medizinprodukt in den Mund, auf die Schleimhäute oder auf die Haut übergehen. Demnach muß das Pigment speichel- und schweißecht sein.

Nach DIN 71-3:1994 unterliegt der Schwermetallgehalt einer gewissen Obergrenze. Da aber nicht ausgeschlossen werden kann, daß das erfindungsgemäße Pflaster in den Mund von Kleinkindern gelangen kann, sollte auch die EN 648:1993, die eine Ergänzung der DIN 71-3 darstellt, eingehalten werden, wie es in Kapitel XLVII der BGVV-Empfehlung "Spielwaren aus Kunststoffen und anderen Polymeren sowie aus Papier, Karton und Pappe" für Spielzeuge empfohlen wird, die bestimmungsgemäß oder voraussehbar in den Mund genommen werden.

Schließlich sollen die Pigmente den europäischen Bestimmungen zur Sicherheit von Spielzeugen genügen (EU Richtlinie 88/378/EG).

Um die aufgeführten Anforderungen sicher einzuhalten, sollte die obere Seite des Trägermaterials, auf der sich der langnachleuchtende Aufdruck befindet, mit einer mechanischen Barriere versehen sein. Vorzugsweise handelt es sich dabei um eine Kunststoffolie oder eine Lackschicht, beispielsweise ein kationisch härtender UV-Flexodrucklack. Alternativ kann diese Schutzschicht auch dadurch erzeugt werden, daß nach dem Konterdruckverfahren das Druckbild des langnachleuchtenden Aufdrucks spiegelverkehrt aufgebracht und anschließend einem Laminat zukaschiert wird.

Weiterhin vorzugsweise ist das Lacksystem mit einem Verdicker abgemischt, wobei der Anteil des Verdickers bezogen auf das Lacksystem zwischen 1 Gew.-% und 20 Gew.-%, insbesondere zwischen 2 Gew.-% und 10 Gew.-% liegen kann. Ein möglicher Verdicker ist beispielsweise die Tixpaste UV (Artikel-Nr. 78-2-028) der Firma SICPA Druckfarben GmbH, Backnang.

Bei dem Verdicker kann es sich handeln um einen mineralischen Verdicker, zum Beispiel eine kolloidale Kieselsäure, oder um einen Verdicker mit einer Polymerstruktur, beispielsweise ein silikonbasierter Verdicker, der die Eigenschaft aufweist, dreidimensional zu vemetzen.

Der in dem Lacksystem befindliche Verdicker verhindert oder verlangsamt durch eine entsprechende Veränderung der Viskosität nach dem Stokes'schen Gesetz ein Sedimentieren der Pigmente. Der Verdicker sorgt somit für eine Steigerung des Nachleuchteffekts des Lacksystems. Ein Maximum an Nachleuchtintensität ist zwischen 2 Gew.-% und 10 Gew.-% Verdicker in dem Lacksystem anzutreffen.

Beim Zusatz höherer Mengen an Verdicker zum Lacksystem, und zwar Anteile von 20 Gew.-% und mehr, ist davon auszugehen, daß der Verdicker die Pigmente einhüllt, so daß der Nachleuchteffekt des Lacksystems getrübt wird.

Des weiteren wird von der Erfindung auch das Verfahren zur Herstellung eines Pflasters umfaßt. Das Verfahren besteht dabei aus folgenden Verfahrensschritten:
a) zumindest einmaliges vollflächiges Bedrucken der oberen Seite des Trägermaterials mit weißer Flexodruckfarbe, um die Nachleuchtintensität der Pigmente zu erhöhen und gleichzeitig Opazitätseigenschaften an dem fertigen Produkt zu erzielen,
b) gegebenenfalls Bedrucken der im Verfahrensschritt d) nicht zu bedruckenden Teilbereiche der oberen Seite des Trägermaterials im Flexodruckverfahren,
c) Herstellung der Mischung aus einem transparenten Lacksystem und dem nachleuchtenden Pigment,
d) Bedrucken der oberen Seite des Trägermaterials mit der Mischung vorzugsweise im Siebdruck, bei partiellem Bedrucken Füllung der im Verfahrensschritt b) ausgesparten weißen Bereiche über ein Siebwerk,
e) gegebenenfalls Nachvemetzen der Mischung mittels UV-Strahlung oder thermisch.

Das im Verfahrensschritt a) erfolgende Bedrucken kann nach allen üblichen Varianten erfolgen. So ist beispielsweise ein Flexodruck, ein Siebdruck oder ein Tiefdruck möglich. Neben dem in Verfahrensschritt d) aufgeführten Siebdruck kann die Mischung aber auch im Rasterflexodruck oder Tiefdruck aufgebracht werden.

Bei partiellem Auftrag des mit dem langnachleuchtenden Pigments abgemischten Lacksystems auf der oberen Seite des Trägermaterials ist nahezu jede beliebige Vorlage denkbar. Die Auswahl der Motive kann sich dabei an dem Alter der jeweiligen Zielgruppe für das erfindungsgemäße Pflaster orientieren.
Für Kleinkinder sind Abbildungen von Puppen, Teddys, Sterne, Sonnen oder ähnlichem vorstellbar, für Kinder bekannte Figuren aus der weiten Welt der Comics, zum Beispiel Asterix oder Obelix, aber auch Figuren aus dem Umkreis von Donald Duck und Micky Maus, oder allgemein dem jeweiligen Zeitgeschmack entsprechende Motive, beispielsweise Dinosaurier, des weiteren einfach Darstellungen von Pflanzen, Tieren oder Menschen.

Diese aufgeführten Beispiele stellen aber nur einen kleinen Ausschnitt aus dem fast unbegrenzten Bereich der Möglichkeiten dar. Dem Fachmann stehen vielfältige Möglichkeiten offen, das erfindungsgemäße Pflaster in seiner Ausführung dem jeweils gewünschten Zweck bzw. der jeweils gewünschten Zielgruppe anzupassen. Weitere Ausführungsarten lassen sich durch die Auswahl eines ein- oder mehrfarbig gefärbten Trägermaterials gewinnen, so daß eine gezielte Gestaltung des Pflasters für ein optimales Aussehen möglich ist.

Eine zusätzliche Steigerung der Variationsmöglichkeiten ist dadurch erzielbar, daß die mit dem langnachleuchtenden Aufdruck nicht versehenen Flächen in beliebigen Farben einfarbig oder bunt bedruckt werden, wobei die Farbauswahl gezielt auf die gewünschten Motive abgestimmt werden kann.

Die äußeren Ausmaße des erfindungsgemäßen Pflasters bewegen sich im Rahmen heute üblicherweise verwendeter Pflaster. In einer bevorzugten Ausführungsform weist die horizontale Achse des Pflasters eine Länge von 10 mm bis 40 mm auf, die vertikale Achse eine Länge von 40 mm bis 80 mm.

Erfindungsgemäße Pflaster verlieren in den Augen der Jugendlichen ihren eigentlichen Charakter als Pflaster. Sie vermögen den Eindruck eines beliebten Stickers oder gar einer Art Tätowierung hervorzurufen. Die Abneigung der Kinder und Jugendlichen gegen derartige Produkte ist sehr gering, so daß die Verwendung erfindungsgemäßer Pflaster neben dem positiven Einfluß auf den Verlauf der Wundheilung gleichzeitig eine Art Schmuckcharakter erzielt.

Im folgenden soll anhand eines Beispiels der Herstellungsprozeß eines erfindungsgemäßen Pflasters dargelegt werden, ohne in irgendeiner Form einschränkend wirken zu sollen.

### Beispiel

Zur Herstellung der Pflaster wurde zunächst das Trägermaterial, eine 60 um dicke, transparente Polyethylen-Pflasterfolie, mit einer hautverträglichen Klebstoffschicht auf Basis vernetzter Polyacrylsäure-Derivate beschichtet, mit einer Wundauflage versehen und mit einem die Klebstoffschicht abdeckenden, silikonisierten Papier kaschiert. Eine Klebemasse des genannten Typs ist in der Schrift DE 27 43 979 C3 dargelegt.

Die obere Seite des Trägermaterials wurde zweimal vollflächig mit weißer Flexodruckfarbe (67S 826309 der Firma Hostmann-Steinberg GmbH) im Flexodruck bedruckt.

Anschließend wurden die Flächenbereiche des Trägermaterials, die nicht mit langnachleuchtenden Motiven versehen werden sollten, mit blauer Flexodruckfarbe (63S 830809 der Firma Hostmann-Steinberg GmbH) ebenfalls im Flexodruckverfahren bedruckt.

Parallel wurde eine Mischung aus dem Pigmentpulver "UV Transparent Nachleuchtfarbe" (Artikel-Nr. 78-2-133) und dem Lack "UV Transparent Nachleuchtfarbe" (Artikel-Nr. 78-2-133-1), jeweils von der Firma SICPA Druckfarben GmbH, Backnang, hergestellt. Dieses transparente Lacksystem wurde dann mit dem langnachleuchtenden Pigment Lumilux ® Effekt grün N-L von Riedel-de Häen vermischt, und zwar im Mengenverhältnis 1:1.

Diesem System wurde weiterhin der Verdicker "Tixpaste UV" (Artikel-Nr. 78-2-028) der Firma SICPA Druckfarben GmbH, Backnang, zugesetzt, und zwar zu einem Anteil von 5 Gew.-% bezogen auf das System.

Die vorher ausgesparten Flächen wurden dann mittels Verwendung eines Spezialsiebs (mit einer Maschenweite von 77 mesh bei 40%igem offenem Anteil) mit der Mischung versehen, was gezielte Auftragen an den Stellen ermöglicht, an denen das Nachleuchten erwünscht ist, und dies bei sehr hoher Auftragsmenge der Mischung.

Anschließend wurden mittels einer rotativen Stanze die Pflaster ausgestanzt, in Papier eingesiegelt, um das Pflaster gegen äußere Verschmutzung oder Kontamination zu schützen, und abschließend zu einer Rolle aufgewickelt.

## Patentansprüche

1. Pflaster zum Verkleben auf der Haut, insbesondere zur Abdeckung von kleineren Wunden, bestehend aus einem Trägermaterial, das auf der unteren Seite mit einer hautverträglichen selbstklebenden Schicht versehen ist,
**dadurch gekennzeichnet, daß**
auf der oberen Seite des Trägermaterials vollflächig oder partiell ein langnachleuchtender Aufdruck vorhanden ist, bestehend aus einem Lacksystem, in das ein langnachleuchtendes Pigment eingearbeitet ist.

2. Pflaster nach Anspruch 1, **dadurch gekennzeichnet, daß** das Trägermaterial aus einer Polyethylenfolie besteht.

3. Pflaster nach einem der Ansprüche 1 oder 2, **dadurch gekennzeichnet, daß** das Trägermaterial weiß eingefärbt und/oder weiß unterlegt oder bedruckt ist.

4. Pflaster nach einem der Ansprüche 1 bis 3, **dadurch gekennzeichnet, daß** auf der klebenden Seite des Trägermaterials eine Wundauflage aufgebracht ist, die kleiner als die Klebefläche ist.

5. Pflaster nach einem der Ansprüche 1 bis 4, **dadurch gekennzeichnet, daß** die Wundauflage in der Mitte des Trägermaterials aufgebracht ist.

6. Pflaster nach einem der Ansprüche 1 bis 5, **dadurch gekennzeichnet, daß** die selbstklebend ausgerüstete Seite des Trägermaterials mit mindestens einem abziehbaren Deckblatt als Schutzabdeckung versehen ist.

7. Pflaster nach einem der Ansprüche 1 bis 6, **dadurch gekennzeichnet, daß** das Pigment ein langnachleuchtendes Pigment aus der Gruppe Lumilux ® N ist.

8. Pflaster nach einem der Ansprüche 1 bis 7, **dadurch gekennzeichnet, daß** das langnachleuchtende Pigment auf einer mit Kupferionen dotierten Zinksulfidverbindung basiert, bevorzugt Lumilux ® Effekt grün N-L.

9. Pflaster nach einem der Ansprüche 1 bis 8, **dadurch gekennzeichnet, daß** dem Lacksystem ein Verdicker zugesetzt ist.

10. Pflaster nach einem der Ansprüche 1 bis 9, **dadurch gekennzeichnet, daß** die obere Seite des Trägermaterials samt des langnachleuchtenden Aufdrucks mit einer Kunststoffolie oder einer Lackschicht überzogen ist.

11. Pflaster nach einem der Ansprüche 1 bis 10, **dadurch gekennzeichnet, daß** die geometrische Form des Trägermaterials mit den äußeren Konturen des auf der oberen Seite des Trägermaterials befindlichem Aufdruck im wesentlichen übereinstimmt.

12. Verfahren zur Herstellung eines Pflasters nach mindestens einem der vorhergehenden Ansprüche, bestehend aus den folgenden Verfahrensschritten:
a) zumindest einmaliges vollflächiges Bedrucken der oberen Seite des Trägermaterials mit weißer Flexodruckfarbe im Flexodruck oder Siebdruck,
b) gegebenenfalls Bedrucken der im Verfahrensschritt d) nicht zu bedruckenden Teilbereiche der oberen Seite des Trägermaterials im Flexodruckverfahren,
c) Herstellung der Mischung aus einem Lacksystem und dem nachleuchtenden Pigment,
d) Bedrucken der oberen Seite des Trägermaterials mit der Mischung vorzugsweise im Siebdruck,
bei partiellem Bedrucken Füllung der im Verfahrensschritt b) ausgesparten weißen Bereiche über ein Siebwerk,
e) gegebenenfalls Nachvemetzen der Mischung mittels UV-Strahlung oder thermisch.

## Claims

1. Plaster for adhesive bonding to the skin, in particular for the covering of relatively small wounds, consisting of a support material which is provided on its bottom side with a dermatologically compatible self-adhesive layer, **characterized in that** all or part of the top side of the support material bears a long-afterglow imprint consisting of a coating system into which a long-afterglow pigment has been incorporated.

2. Plaster according to Claim 1, **characterized in that** the support material consists of a polyethylene film.

3. Plaster according to one of Claims 1 and 2, **characterized in that** the support material is white in coloration and/or has a white underlay or imprint.

4. Plaster according to one of Claims 1 to 3, **characterized in that** a wound pad which is smaller than the bond surface is applied to the adhesive side of the support material.

5. Plaster according to one of Claims 1 to 4, **characterized in that** the wound pad is applied in the centre of the support material.

6. Plaster according to one of Claims 1 to 5, **characterized in that** the support material side which is rendered self-adhesive is provided with at least one tear-off cover sheet as a protective covering.

7. Plaster according to one of Claims 1 to 6, **characterized in that** the pigment is a long-afterglow pigment from the Lumilux® N group.

8. Plaster according to one of Claims 1 to 7, **characterized in that** the long-afterglow pigment is based on a zinc sulphide compound doped with copper ions, preferably Lumilux® Effekt grün N-L.

9. Plaster according to one of Claims 1 to 8, **characterized in that** a thickener has been added to the coating system.

10. Plaster according to one of Claims 1 to 9, **characterized in that** the top side of the support material, including the long-afterglow imprint, is coated with a plastic film or a varnish coat.

11. Plaster according to one of Claims 1 to 10, **characterized in that** the geometric shape of the support material substantially coincides with the outer contours of the imprint situated on the top side of the support material.

12. Process for producing a plaster according to at least one of the preceding claims, consisting of the following steps:
a) at least one printing of the top side of the support material over its full area with white flexographic printing ink by flexographic printing or screen printing,
b) if desired, printing of the part-areas of the top side of the support material that are not to be printed in step d), by flexographic printing,
c) preparing the mixture comprising a coating system and the afterglow pigment,
d) printing the top side of the support material with the mixture, preferably by screen printing,
in the case of partial printing using a screen mechanism to fill in the white areas defined in step b), and
e) if desired, aftercrosslinking the mixture by means of UV radiation, or thermally.

## Revendications

1. Pansement destiné à être collé sur la peau, en particulier pour le recouvrement de petites plaies, constitué d'un matériau support qui est pourvu sur sa face inférieure d'une couche autoadhésive supportée par la peau, **caractérisé en ce qu'**une impression phosphorescente est présente sur toute la face supérieure ou sur une partie de la surface supérieure du matériau support, constituée d'un système de vernis dans lequel est incorporé un pigment phosphorescent.

2. Pansement selon la revendication 1, **caractérisé en ce que** le matériau support est constitué d'un film en polyéthylène.

3. Pansement selon l'une quelconque des revendications 1 ou 2, **caractérisé en ce que** le matériau support est teinté de blanc et/ou présente un substrat blanc ou est imprimé de blanc.

4. Pansement selon l'une quelconque des revendications 1 à 3, **caractérisé en ce qu'**une couche pour plaie est appliquée sur la face adhésive du matériau support, qui est plus petite que la face adhésive.

5. Pansement selon l'une quelconque des revendications 1 à 4, **caractérisé en ce que** la couche pour plaie est appliquée au milieu du matériau support.

6. Pansement selon l'une quelconque des revendications 1 à 5, **caractérisé en ce que** la face pourvue de manière autoadhésive du matériau support est pourvue d'au moins une feuille de recouvrement pouvant être retirée comme revêtement de protection.

7. Pansement selon l'une quelconque des revendications 1 à 6, **caractérisé en ce que** le pigment est un pigment phosphorescent du groupe Lumilux® N.

8. Pansement selon l'une quelconque des revendications 1 à 7, **caractérisé en ce que** le pigment phosphorescent est basé sur un composé sulfure de zinc dopé d'ions de cuivre, de préférence le Lumilux® Effekt grün N-L.

9. Pansement selon l'une quelconque des revendications 1 à 8, **caractérisé en ce qu'**un épaississant est mélangé au système de vernis.

10. Pansement selon l'une quelconque des revendications 1 à 9, **caractérisé en ce que** la face supérieure du matériau support avec l'impression phosphorescente est recouverte d'un film de matériau synthétique ou d'une couche de vernis.

11. Pansement selon l'une quelconque des revendications 1 à 10, **caractérisé en ce que** la forme géométrique du matériau support correspond essentiellement aux contours extérieurs de l'impression se trouvant sur la face supérieure du matériau support.

12. Procédé pour la fabrication d'un pansement selon au moins l'une quelconque des revendications précédentes, constitué des étapes de procédé suivantes :
a) au moins une impression unique sur toute la surface de la face supérieure du matériau support avec une encre de flexographie blanche par flexographie ou sérigraphie,
b) le cas échéant impression des zones partielles, qui ne seront pas imprimées dans l'étape de procédé d), de la face supérieure du matériau support dans un procédé de flexographie,
c) préparation du mélange d'un système de vernis et du pigment phosphorescent,
d) impression de la face supérieure du matériau support avec le mélange, de préférence par sérigraphie,
dans le cas d'une impression partielle, remplissage des zones blanches laissées à nu dans l'étape de procédé b) via un pochoir de sérigraphie,
e) le cas échéant post-réticulation du mélange au moyen d'un rayonnement UV ou par voie thermique.
